(19) Europäisches Patentamt
European Patent Office
Office européen des brevets

(11) EP 2 794 014 B1

(12) **EUROPÄISCHE PATENTSCHRIFT**

(45) Veröffentlichungstag und Bekanntmachung des Hinweises auf die Patenterteilung:
**02.01.2019 Patentblatt 2019/01**

(21) Anmeldenummer: **12783234.3**

(22) Anmeldetag: **09.11.2012**

(51) Int Cl.:
***A61Q 5/06*** *(2006.01)* ***A61K 8/81*** *(2006.01)*
***A61K 8/41*** *(2006.01)* ***A61K 8/898*** *(2006.01)*

(86) Internationale Anmeldenummer:
**PCT/EP2012/072278**

(87) Internationale Veröffentlichungsnummer:
**WO 2013/091996 (27.06.2013 Gazette 2013/26)**

(54) **MITTEL ZUR TEMPORÄREN VERFORMUNG KERATINISCHER FASERN AUF GRUNDLAGE EINER KOMBINATION SPEZIFISCHER FILMBILDENDER POLYMERE**

PRODUCT FOR TEMPORARILY SHAPING KERATIN FIBERS ON THE BASIS OF A COMBINATION OF SPECIFIC FILM-FORMING POLYMERS

PRODUIT DE MISE EN FORME TEMPORAIRE DE FIBRES KÉRATINIQUES À BASE D'UNE ASSOCIATION DE POLYMÈRES FILMOGÈNES SPÉCIFIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2011 DE 102011089169**

(43) Veröffentlichungstag der Anmeldung:
**29.10.2014 Patentblatt 2014/44**

(73) Patentinhaber: **Henkel AG & Co. KGaA**
**40589 Düsseldorf (DE)**

(72) Erfinder:
- **METTEN, Diane**
  **22393 Hamburg (DE)**
- **RICHTERS, Bernd**
  **21129 Hamburg (DE)**
- **SCHEFFLER, Rene**
  **25470 Ellerau (DE)**

(56) Entgegenhaltungen:
**EP-A1- 2 335 673** **DE-A1-102006 045 964**
**JP-A- 2002 145 725** **US-A1- 2007 134 191**

- **anonymous: "FLEXAN II", AkzoNobel , XP002711705, Gefunden im Internet: URL:http://sc.akzonobel.com/en/personalcare/Pages/product-detail.aspx?prodID=6681 [gefunden am 2013-08-16]**
- **HÖSSEL P ET AL: "Polyquaternium-68: Mehr als Haarfestigung", SOFW-JOURNAL( SEIFEN,OELE,FETTE,WACHSE ), VERLAG F}R CHEMISCHE INDUSTRIE , Bd. 131 1. Mai 2005 (2005-05-01), Seiten 19-29, XP002540039, AUGSBURG, DE Gefunden im Internet: URL:http://www.sofw.com/index/sofw_de/sofw_de_archiv.html?cosearch=polyquaternium-68&cosearch_sa=polyquaternium-68&costart=21&date_from[Y]=2005&date_from[m]=4&date_until[Y]=2005&date_until[m]=7&do_search_sa=1&naid=604 [gefunden am 2009-08-05]**
- **DATABASE GNPD [Online] MINTEL; Februar 2008 (2008-02), "Aerostraight Spray-In Strightener", XP002711706, Database accession no. 863554**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 794 014 B1

## Beschreibung

Die vorliegende Anmeldung beschreibt kosmetische Mittel auf Grundlage einer spezifischen Polymerkombination, die Verwendung dieser kosmetischen Mittel zur temporären Verformung keratinischer Fasern sowie kosmetische Verfahren unter Einsatz dieser Mittel.

Der Einsatz von Polymeren in verschiedensten kosmetischen Mitteln ist weit verbreitet. Sie finden sich in Mitteln zur Behandlung der Haut ebenso wie in Mitteln zur Behandlung der Haare, in Mitteln, die unmittelbar nach der Anwendung wieder ab- bzw. ausgewaschen werden, so genannten Rinse-off Produkten, genauso wie in Mitteln, die auf Haut oder Haar verbleiben, so genannten Leave-on Mitteln. Dabei werden die Polymere aus unterschiedlichsten Gründen eingesetzt und jeweils bestimmte Eigenschaften der Polymere ausgenutzt. In Mitteln zur Hautbehandlung, in Shampoos, Haarspülungen und Haarkuren stehen oftmals die verdickenden oder pflegenden Eigenschaften der Polymere im Vordergrund. In Mitteln zur temporären Verformung keratinischer Fasern, im Folgenden auch Stylingmittel genannt, sind neben diesen Eigenschaften vor allem filmbildende und/oder festigende Wirkungen gefragt. Oftmals dienen Polymere auch als Hilfsmittel um die Abscheidung und Fixierung anderer Wirk- und Inhaltsstoffe auf der Haut oder dem Haar zu verbessern oder erst möglich zu machen. So lassen sich beispielsweise durch Zusatz geeigneter Polymere zu Haarfärbemitteln die Reibechtheiten und die Beständigkeit der Färbung erhöhen.

In der Regel enthalten kosmetische Mittel einzelne Polymere, die speziell darauf zugeschnitten sind, einen ganz bestimmten Effekt zu erzielen. Sollen verschiedene Effekte erzielt werden, ist die Zugabe mehrerer Polymere erforderlich. Werden allerdings zu viele verschiedene Polymere eingesetzt, kann das eine Reihe von Nachteilen mit sich bringen. So können Probleme bei der Formulierung entstehen, etwa weil die Polymere untereinander oder mit anderen Bestandteilen des Mittels reagieren und es zu Ausfällungen oder Zersetzungen kommt. Bestimmte Polymere neigen auch dazu, sich auf der Haut und insbesondere auf dem Haar so beständig abzuscheiden, dass sie bei einer gewöhnlichen Wäsche nicht mehr vollständig entfernt werden und es zu einer unerwünschten Anreicherung des Polymers und damit letztlich einer Belastung von Haut oder Haar kommt.

Es besteht daher ständig Bedarf an Polymeren oder geeigneten Kombinationen weniger Polymere, die möglichst viele der gewünschten Eigenschaften gleichzeitig aufweisen.

Beispielsweise ist es im Falle der Stylingmittel notwendig, dass die eingesetzten Polymeren dem behandelten Haar einen möglichst starken Halt geben. Neben einem hohen Haltegrad müssen Stylingmittel jedoch eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein. Handelt es sich bei dem Stylingmittel um ein Gel oder eine Paste, sollen die Polymere zudem verdickende Eigenschaften besitzen.

Aus der DE 10 2006 045 964 A1 sind Mittel zur temporären Verformung keratinischer Fasern bekannt, welche zwei ausgewählte Copolymere enthalten. Ein Copolymer weist mindestens ein N-Vinyllactam und quaternisiertes N-Vinylimidazol oder N-Vinylimidazol als Monomer auf und kann das Copolymer mit der INCI-Bezeichnung Polyquaternium-68 sein.

Die EP 2335673 A1 beschreibt kosmetische Schäume zur temporären Haarverformung, welche neben anderen Bestandteilen mindestens ein kationisches Polymer enthalten. Ein bevorzugtes kationisches Polymer ist das Polymer mit der INCI-Bezeichnung Polyquaternium-68.

Die Aufgabe der vorliegenden Erfindung war es daher, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie stabil transparenter kosmetischer Zusammensetzungen eignen.

Diese Aufgaben wurden durch eine spezielle Polymerkombination gelöst. Ein erster Gegenstand der Erfindung ist daher ein kosmetisches Mittel, enthaltend in einem kosmetisch akzeptablen Träger

a) mindestens ein Polystyrolsulfonat

b) mindestens ein Copolymer B gebildet aus

- mindestens einem Monomer (B1) ausgewählt aus quaternisierten N-Vinylimidazolen
- mindestens einem Monomer (B2) ausgewählt aus N-Vinyllactamen,

wobei das Gewichtsverhältnis von Polystyrolsulfonat und B zwischen 4:1 und 1:4 beträgt.

Die erfindungsgemäßen Mittel enthalten die Wirkstoffe in einem kosmetischen Träger. Dieser kosmetische

Träger ist im Sinne der Erfindung ist wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Bevorzugte kosmetische Mittel enthalten, bezogen auf sein Gesamtgewicht, 40 bis 99 Gew.-%, vorzugsweise 50 bis 98 Gew.-%, besonders bevorzugt 60 bis 95 Gew.-% und insbesondere 70 bis 90 Gew.-% Wasser. Der pH-Wert (10%-ige Lösung, 20°C) bevorzugter kosmetischer Mittel beträgt 4 bis 9, vorzugsweise 5 bis 8 und insbesondere von 6 bis 7.

Als ersten wesentlichen Bestandteil enthalten die erfindungsgemäßen kosmetischen Mittel mindestens ein Polystyrolsulfonat A. Bevorzugte Polystyrolsulfonat zeichnen sich durch ein hohes Molukulargewicht aus. Bevorzugt werden insbesondere Polystyrolsulfonat A, die ein Molukulargewicht von 80 bis 180 kDa, vorzugsweise von 100 bis 160 kDa und insbesondere von 120 bis 140 kDa aufweisen.

Der Gewichtsanteil des Copolymers A am Gesamtgewicht erfindungsgemäßer kosmetischer Mittel beträgt bevorzugt 0,05 bis 10 Gew.-%, vorzugsweise 0,1 bis 7,0 Gew.-% und insbesondere 0,2 bis 5,0 Gew.-%.

Die erfindungsgemäßen Mittel enthaltend als zweiten wesentlichen Bestandteil mindestens ein Copolymer B gebildet aus

- mindestens einem Monomer (B1) ausgewählt aus quaternisierten N-Vinylimidazolen.
- mindestens einem Monomer (B2) ausgewählt aus N-Vinyllactamen,

Besonders bevorzugte erfindungsgemäße Mittel sind dadurch gekennzeichnet, dass sie mindestens ein Copolymer B enthalten, das

- mindestens eine Struktureinheit gemäß Formel (B-I) enthält, worin
    - R eine $C_1$- bis $C_{30}$-Alkylgruppe, eine $C_1$- bis $C_4$-Aralkylgruppe, eine $C_2$- bis $C_6$-Alkenylgruppe oder eine $C_2$ bis $C_6$-Hydroxyalkylgruppe bedeutet und
    - $X^-$ für ein physiologisch verträgliches Anion steht
- und mindestens eine weitere Struktureinheit gemäß Formel (B-II) enthält, worin n für 1, 2 oder 3 als Anzahl der Methyleneinheiten steht.

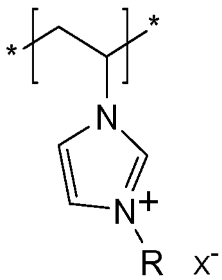 (B-I) 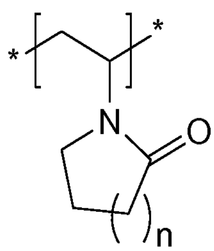 (B-II)

Filmbildende und/oder festigende Copolymere B sind bekannt. Diese Copolymere besitzen mindestens eine Struktureinheit gemäß Formel (B-I) und mindestens eine Struktureinheit gemäß Formel (B-II) und können darüber hinaus weitere Struktureinheiten aufweisen, die durch das Hinzufügen entsprechender Monomere bei der Polymerisation einpolymerisiert werden.

In Formel (B-I) steht R für eine $C_1$- bis $C_{30}$-Alkylgruppe, eine $C_1$- bis $C_4$-Aralkylgruppe, eine $C_2$- bis $C_6$-Alkenylgruppe oder eine $C_2$ bis $C_6$-Hydroxyalkylgruppe. Bevorzugte Gruppen R sind beispielsweise $-CH_3$; $-CH_2CH_3$, $-CH_2CH_2CH_3$, $CH(CH_3)_2$, $-(CH_2)_3CH_3$, $-CH_2-CH(CH_3)_2$, $CH(CH_3)CH_2CH_3$, $-C(CH_3)_3$, $-CH_2OH$, $-CH_2CH_2OH$, $-CH_2CH_2CH_2OH$, $-CH(OH)CH_2CH_3$, $-CH_2CH(OH)CH_3$.

$X^-$ steht für ein physiologisch verträgliches Anion, bevorzugte Anionen sind Chlorid, Bromid, Iodid, Sulfat, Methosulfat, Ethylsulfat, Tosylat und Tetrafluoroborat. Besonders bevorzugte Mittel sind dadurch gekennzeichnet, dass es sich bei dem Monomer (B1) um ein Salz von 3-Alkyl-1-vinylimidazolium mit physiologisch verträglichen Anionen, vorzugsweise um 3-Methyl-1-vinylimidazoliummethylsulfat handelt.

In Formel (B-II) steht n für die Anzahl der Methylengruppen. Mit n = 1 steht Formel (B-II) für eine Vinylpyrrolidon-Einheit, mit n = 2 für eine Vinylpiperidinon-Einheit und mit n = 3 für eine Vinylcaprolactam-Einheit. Besonders bevorzugte

Mittel sind dadurch gekennzeichnet, dass Monomer (B2) ausgewählt ist aus N-Vinylcaprolactam und N-Vinylpyrrolidon. Ganz besonders bevorzugt wird als Monomer (B2) N-Vinylpyrrolidon eingesetzt.

Besonders bevorzugte erfindungsgemäße Mittel sind dadurch gekennzeichnet, dass sie als Copolymer B ein Copolymer B1 enthalten, das

- mindestens eine Struktureinheit gemäß Formel (B-I) enthält, worin R für eine Methylgruppe und X für Methosulfat steht,
- mindestens weitere Struktureinheit gemäß Formel (B-II) enthält, worin n für 1 Methyleneinheiten steht.

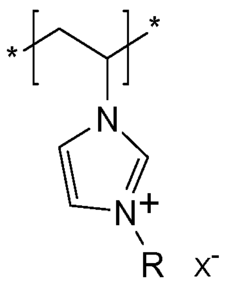 (B-I)

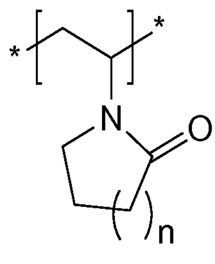 (B-II)

Ganz besonders bevorzugte Copolymere B1 enthalten 10 bis 30 Mol-%, vorzugsweise 15 bis 25 Mol.-% und insbesondere 20 Mol.-% Struktureinheiten gemäß Formel (B-I) und 70 bis 90 Mol.-%, vorzugsweise 75 bis 85 Mol.-% und insbesondere 80 Mol.-% Struktureinheiten gemäß Formel (B-II).

Hierbei ist besonders bevorzugt, wenn die Copolymere B1 neben Polymereinheiten, die aus dem Einbau der genannten Struktureinheiten gemäß Formel (B-I) und (B-II) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere B1 ausschließlich aus Struktureinheiten der Formel (B-I) und (B-II) aufgebaut und lassen sich durch die allgemeine Formel

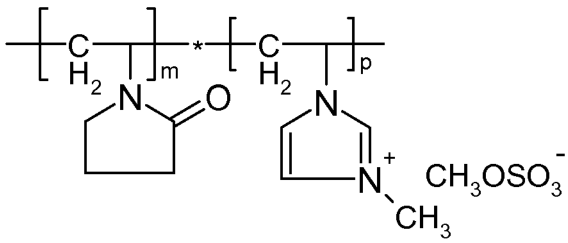

beschreiben, wobei die Indices m und n je nach Molmasse des Polymers variieren und nicht bedeuten sollen, dass es sich um Blockcopolymere handelt. Vielmehr können Struktureinheiten der Formel (B-I) und der Formel (B-II) im Molekül statistisch verteilt vorliegen.

Solche N-Methylvinylimidazol/Vinylpyrrolidon-Copolymere werden laut INCI-Nomenklatur als POLYQUATERNIUM-44 bezeichnet und sind beispielsweise von der BASF unter dem Handelsnamen Luviquat® UltraCare erhältlich.

Besonders bevorzugte erfindungsgemäße Mittel enthalten ein Copolymer B1, das Molmassen innerhalb eines bestimmten Bereiches aufweist. Hier sind Haarverformungsmittel bevorzugt, bei denen das Copolymer B1 eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) B1 oder an dessen bzw. deren Stelle können die erfindungsgemäßen Mittel I auch Copolymere B2 enthalten, die als zusätzliche Struktureinheiten der Formel (B-II) aufweisen, in der n für die Zahl 3 steht.

Weitere besonders bevorzugte Mittel sind dadurch gekennzeichnet, dass sie als Copolymer B ein Copolymer B2 enthalten, das

- mindestens eine Struktureinheit gemäß Formel (B-I) enthält. worin R für eine Methylgruppe und X für Methosulfat steht,
- mindestens weitere Struktureinheit gemäß Formel (B-II) enthält, worin n für 1 Methyleneinheiten steht
- mindestens weitere Struktureinheit gemäß Formel (B-II) enthält, worin n für 3 Methyleneinheiten steht.

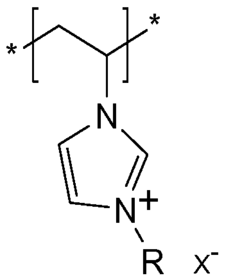 (B-I) 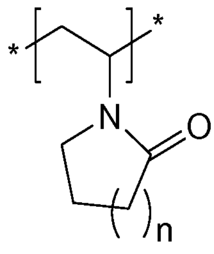 (B-II) 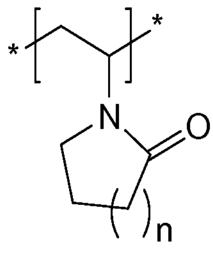 (B-II)

Auch hierbei ist besonders bevorzugt, wenn die Copolymere B2 neben Polymereinheiten, die aus dem Einbau der genannten Struktureinheiten gemäß Formel (B-I) und (B-II) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere B2 ausschließlich aus Struktureinheiten der Formel (B-I) und (B-II) aufgebaut und lassen sich durch die allgemeine Formel

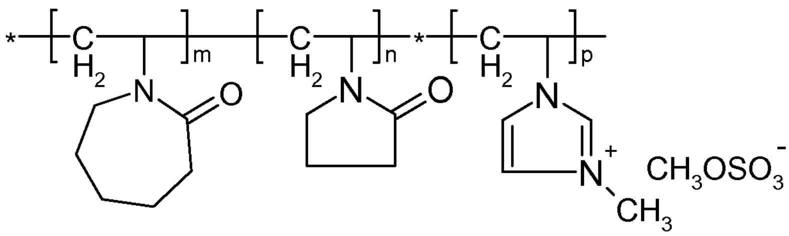

beschreiben, wobei die Indices m, n und p je nach Molmasse des Polymers variieren und nicht bedeuten sollen, dass es sich um Blockcopolymere handelt. Vielmehr können Struktureinheiten der Formel (B-I) und der Formel (B-II) im Molekül statistisch verteilt vorliegen.

Solche N-Methylvinylimidazol/Vinylpyrrolidon/Vinylcaprolactam-Copolymere werden laut INCI-Nomenklatur als POLYQUATERNIUM-46 bezeichnet und sind beispielsweise von der BASF unter dem Handelsnamen Luviquat® Hold erhältlich.

Ganz besonders bevorzugte Copolymere B2 enthalten 1 bis 20 Mol-%, vorzugsweise 5 bis 15 Mol.-% und insbesondere 10 Mol.-% Struktureinheiten gemäß Formel (B-I) und 30 bis 50 Mol.-%, vorzugsweise 35 bis 45 Mol.-% und insbesondere 40 Mol.-% Struktureinheiten gemäß Formel (B-II) mit n = 1 und 40 bis 60 Mol.-%, vorzugsweise 45 bis 55 Mol.-% und insbesondere 60 Mol.-% Struktureinheiten gemäß Formel (B-II) mit n = 3.

Besonders bevorzugte Mittel enthalten ein Copolymer B2, das Molmassen innerhalb eines bestimmten Bereiches aufweist. Hier sind Haarverformungsmittel bevorzugt, bei denen das Copolymer B2 eine Molmasse von 100 bis 1000 kDa, vorzugsweise von 250 bis 900 kDa, weiter bevorzugt von 500 bis 850 kDa und insbesondere von 650 bis 710 kDa aufweist.

Zusätzlich zu dem bzw. den Copolymer(en) B1 und/oder B2 oder an dessen bzw. deren Stelle können die Mittel auch Copolymere B3 enthalten, die als zusätzliche Struktureinheiten Struktureinheiten der Formel (B-II) aufweisen, in der n für die Zahl 3 steht sowie weitere Distruktureinheiten aus der Gruppe der Vinylimidazol-Einheiten und weitere Struktureinheiten aus der Gruppe der Acrylamid- und/oder Methacrylamid-Einheiten.

Bevorzugte erfindungsgemäße Mittel sind dadurch gekennzeichnet, dass das Copolymer B gebildet aus

- mindestens einem Monomer (B1) ausgewählt aus quaternisierten N-Vinylimidazolen,
- mindestens einem Monomer (B2) ausgewählt aus N-Vinyllactamen,
- dem Monomer N-Vinylimidazol (B3) und
- mindestens einem Monomer (B4) ausgewählt aus Acrylsäureamid, Methacrylsäureamid, N-Alkylacrylsäureamid und N-Alkylmethacrylsäureamid,

Ganz besonders bevorzugte Mittel sind dadurch gekennzeichnet, dass sie als Copolymer B ein Copolymer B3 enthalten, das

- mindestens eine Struktureinheit gemäß Formel (B-I) enthält, worin R für eine Methylgruppe und X für Methosulfat steht,
- mindestens weitere Struktureinheit gemäß Formel (B-II) enthält, worin n für 1 Methyleneinheiten steht

- mindestens weitere Struktureinheit gemäß Formel (B-III) enthält
- mindestens weitere Struktureinheit gemäß Formel (B-IV) enthält

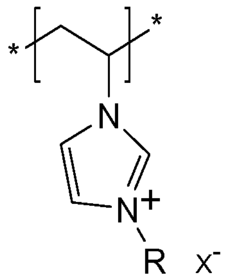 (B-I) 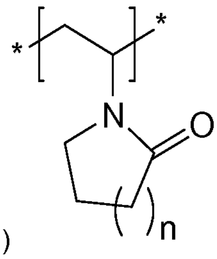 (B-II) 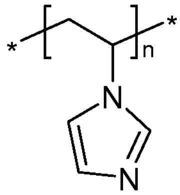 (B-III) 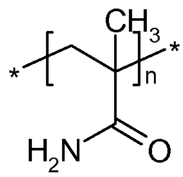 (B-IV).

Auch hierbei ist besonders bevorzugt, wenn die Copolymere B3 neben Polymereinheiten, die aus dem Einbau der genannten Struktureinheiten gemäß Formel (B-I), (B-II), (B-III) und (B-IV) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere B3 ausschließlich aus Struktureinheiten der Formel (B-I), (B-II), (B-III) und (B-IV) aufgebaut und lassen sich durch die allgemeine Formel

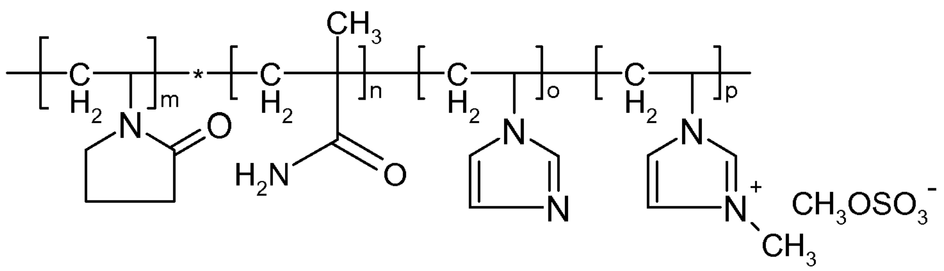

beschreiben, wobei die Indices m, n, o und p je nach Molmasse des Polymers variieren und nicht bedeuten sollen, dass es sich um Blockcopolymere handelt. Vielmehr können Struktureinheiten der Formeln (B-I), (B-II), (B-III) und (B-IV) im Molekül statistisch verteilt vorliegen.

Bevorzugte erfindungsgemäße Mittel sind dadurch gekennzeichnet, dass das Copolymer B gebildet ist aus

- 3-Methyl-1-vinylimidazoliummethylsulfat (B1),
- mindestens einem Monomer (B2) ausgewählt aus N-Vinylcaprolactam und N-Vinylpyrrolidon,
- N-Vinylimidazol (B3) und
- mindestens einem Monomer (B4) ausgewählt aus Acrylsäureamid und Methacrylsäureamid.

Ganz besonders bevorzugt werden erfindungsgemäße Mittel, bei denen das Copolymer B gebildet ist aus

- 3-Methyl-1-vinylimidazoliummethylsulfat (B1),
- N-Vinylpyrrolidon (B2),
- N-Vinylimidazol (B3) und
- Methacrylsäureamid (B4).

In Bezug auf die kosmetischen Eigenschaften der erfindungsgemäßen Mittel hat es sich als vorteilhaft erwiesen, wenn das Copolymer B zu mindestens 70 Gew.-%, vorzugsweise zu mindestens 80 Gew.-%, bevorzugt zu mindestens 90 Gew.-% und insbesondere zu mindestens 95 Gew.-% aus den Struktureinheiten (B1), (B2), (B3) und (B4) besteht.

Solche N-Methylvinylimidazol/Vinylpyrrolidon/Vinylimidazol/Methacrylamid-Copolymere werden laut INCI-Nomenklatur als POLYQUATERNIUM-68 bezeichnet und sind beispielsweise von der BASF unter dem Handelsnamen Luviquat® Supreme erhältlich.

Ganz besonders bevorzugte Copolymere B3 enthalten 1 bis 12 Mol-%, vorzugsweise 3 bis 9 Mol.-% und insbesondere 6 Mol.-% Struktureinheiten gemäß Formel (B-I) und 45 bis 65 Mol.-%, vorzugsweise 50 bis 60 Mol.-% und insbesondere 55 Mol.-% Struktureinheiten gemäß Formel (B-II) mit n = 1 und 1 bis 20 Mol.-%, vorzugsweise 5 bis 15 Mol.-% und insbesondere 10 Mol.-% Struktureinheiten gemäß Formel (B-III) und 20 bis 40 Mol.-%, vorzugsweise 25 bis 35 Mol.-% und insbesondere 29 Mol.-% Struktureinheiten gemäß Formel (B-IV).

Besonders bevorzugte Mittel enthalten ein Copolymer B3, das Molmassen innerhalb eines bestimmten Bereiches aufweist. Hier sind Haarverformungsmittel bevorzugt, bei denen das Copolymer B3 eine Molmasse von 100 bis 500 kDa, vorzugsweise von 150 bis 400 kDa, weiter bevorzugt von 250 bis 350 kDa und insbesondere von 290 bis 310 kDa aufweist.

Der Gewichtsanteil des Copolymers B am Gesamtgewicht erfindungsgemäßer kosmetischer Mittel beträgt bevorzugt 0,05 bis 10 Gew.-%, vorzugsweise 0,1 bis 7,0 Gew.-% und insbesondere 0,2 bis 5,0 Gew.-%.

Die erfindungsgemäßen Mittel zeichnen sich von kosmetischen Mitteln mit alternativen Vinylpyrrolidon Copolymeren neben den oben genannten Vorteilen insbesondere auch durch einen verbesserten Haltegrad aus. Als für die kosmetischen Eigenschaften der erfindungsgemäßen Mittel entscheidend hat sich ein Gewichtsverhältnis von Polystyrolsulfonat und Polymer B in dem kosmetischen Mittel zwischen 4:1 und 1:4 erwiesen.

Das Polystyrolsulfonat wird in dem kosmetischen Mitteln vorzugsweise in teilneutraliserter oder neutralisierter Form eingesetzt. Zur Neutralisation wird bevorzugt mindestens ein Alkanolamin verwendet. Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Als besonders geeignetes Neutralisationsmittel hat sich dabei 2-Amino-2-methylpropanol erwiesen. Erfindungsgemäß bevorzugte kosmetische Mittel enthalten mindestens ein Alkanolamin, vorzugsweise 2-Amino-2-methylpropanol. Das 2-Amino-2-methylpropanol wird in den erfindungsgemäßen Mitteln vorzugsweise in einer Menge eingesetzt, welche die zur Neutralisation des Copolymers A benötigte Menge nicht überschreitet. Vorzugsweise beträgt die in den erfindungsgemäßen Mitteln eingesetzte Mengen an 2-Amino-2-methylpropanol 80 bis 100%, besonders bevorzugt 90 bis 100% und insbesondere 95 bis 100% der zur vollständigen Neutralisation des Copolymers A benötigten Menge. In einer bevorzugten Ausführungsform beträgt der Gewichtsanteil des 2-Amino-2-methylpropanols am Gesamtgewicht des kosmetischen Mittels 0,1 bis 4,0 Gew.-%, bevorzugt 0,2 bis 3,0 Gew.% und insbesondere 0,5 bis 2,0 Gew.-%.

Neben den zuvor beschriebenen Copolymeren und Trägersubstanzen können die erfindungsgemäßen kosmetischen Mittel weitere Inhaltsstoffe enthalten. Zur Gruppe dieser weiteren Inhaltsstoffe zählen insbesondere die kosmetisch wirksamen Hilfs- und Zusatzstoffe.

Als bevorzugten Bestandteil enthalten die erfindungsgemäßen kosmetischen Mittel mindestens eine quartäre Ammoniumverbindung. Als quartäre Ammoniumverbindung können monomere oder polymere Wirkstoffe eingesetzt werden.

Aus der Vielzahl an möglichen monomeren quartären Ammoniumverbindungen haben sich die Verbindungen aus den Gruppen:

- Trimethylalkylammoniumhalogenide;
- der Esterquats
- der quartären Imidazoline

als besonders wirkungsvoll erwiesen.

Zur Gruppe der Trimethylalkylammoniumhalogenide zählen insbesondere die Verbindungen der Formel (Tkat1-1).

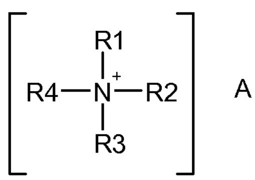

(Tkat1)

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat. Bevorzugte kosmetische Mittel enthalten eine monomere quartäre Ammoniumverbindung aus der Gruppe der Trimethylalkylammoniumhalogenide.

Weitere erfindungsgemäß besonders bevorzugte quartären Ammoniumverbindungen sind die kationischen Betainestern der Formel (Tkat1-2.1).

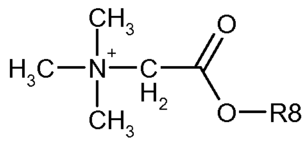

(Tkat1-2.1)

Besonders bevorzugt sind die Esterquats mit den Handelsbezeichnungen Armocare® VGH-70, sowie Dehyquart® F-75, Dehyquart® L80, Stepantex® VS 90 und Akypoquat® 131.

Eine weitere Gruppe sind quartäre Imidazolinverbindungen. Die im Folgenden dargestellte Formel (Tkat2) zeigt die Struktur dieser Verbindungen.

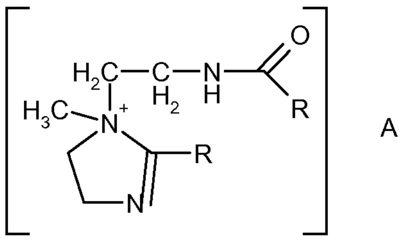

(Tkat2)

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCI - Bezeichnungen Quaternium-27, Quaternium-72, Quaternium-83, Quaternium-87 und Quaternium-91 erhältlich. Höchst bevorzugt ist erfindungsgemäß Quaternium-91.

In Bezug auf die kosmetische Wirkung haben sich kosmetische Mittel als vorteilhaft erwiesen, bei denen der Gewichtsanteil der monomeren quartären Ammoniumverbindung am Gesamtgewicht des Mittels 0,05 bis 3,0 Gew.-%, vorzugsweise 0,1 bis 2,0 Gew.-% und insbesondere 0,2 bis 1,0 Gew.-% beträgt.

Als geeignete Hilfs- und Zusatzstoffe sind insbesondere zusätzliche Pflegestoffe zu nennen.

Als Pflegestoff einer anderen Verbindungsklasse kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte erfindungsgemäße kosmetische Mittel enthalten mindestens einen Ölkörper, vorzugsweise mindestens einen Ölkörper aus der Gruppe der Silikonöle. Zur Gruppe der Silikonöle zählen insbesondere die Dimethicone, zu welchen auch die

Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von $C_6$ - $C_{30}$ - Fettsäuren mit $C_2$ - $C_{30}$ - Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

In Bezug auf die kosmetische Wirkung haben sich kosmetische Mittel als vorteilhaft erwiesen, bei denen der Gewichtsanteil des Ölkörpers am Gesamtgewicht des Mittels 0,01 bis 5,0 Gew.-%, vorzugsweise 0,02 bis 4,0 Gew.-% und insbesondere 0,05 bis 2,0 Gew.-% beträgt.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 | Formel 5 |
|---|---|---|---|---|---|
| Polystyrolsulfonat | 0,05 bis 10 | 0,1 bis 7,0 | 0,2 bis 5,0 | 0,4 | 1,2 |
| Copolymer B | 0,05 bis 10 | 0,1 bis 7,0 | 0,2 bis 5,0 | 0,8 | 0,8 |
| Wasser, Misc | add 100 | add 100 | add 100 | add 100 | add 100 |
| | Formel 6 | Formel 7 | Formel 8 | Formel 9 | Formel 10 |
| Polystyrolsulfonat 1) | 0,05 bis 10 | 0,1 bis 7,0 | 0,2 bis 5,0 | 0,4 | 1,2 |
| Copolymer B | 0,05 bis 10 | 0,1 bis 7,0 | 0,2 bis 5,0 | 0,8 | 0,8 |
| Wasser, Misc | add 100 | add 100 | add 100 | add 100 | add 100 |
| | Formel 11 | Formel 12 | Formel 13 | Formel 14 | Formel 15 |
| Copolymer A | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Wasser, Misc | add 100 | add 100 | add 100 | add 100 | add 100 |
| | Formel 16 | Formel 17 | Formel 18 | Formel 19 | Formel 20 |
| Polystyrolsulfonat 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Wasser, Misc | add 100 | add 100 | add 100 | add 100 | add 100 |
| | Formel 21 | Formel 22 | Formel 23 | Formel 24 | Formel 25 |
| Polystyrolsulfonat | 0,05 bis 10 | 0,1 bis 7,0 | 0,2 bis 5,0 | 0,4 | 1,2 |
| Copolymer B | 0,05 bis 10 | 0,1 bis 7,0 | 0,2 bis 5,0 | 0,8 | 0,8 |
| Alkanolamin 3) | 80 bis 100 | 90 bis 100 | 95 bis 100 | 98 | 98 |
| Wasser, Misc | add 100 | add 100 | add 100 | add 100 | add 100 |
| | Formel 26 | Formel 27 | Formel 28 | Formel 29 | Formel 30 |
| Polystyrolsulfonat 1) | 0,05 bis 10 | 0,1 bis 7,0 | 0,2 bis 5,0 | 0,4 | 1,2 |
| Copolymer B | 0,05 bis 10 | 0,1 bis 7,0 | 0,2 bis 5,0 | 0,8 | 0,8 |

(fortgesetzt)

| | Formel 26 | Formel 27 | Formel 28 | Formel 29 | Formel 30 |
|---|---|---|---|---|---|
| Alkanolamin 3) | 80 bis 100 | 90 bis 100 | 95 bis 100 | 98 | 98 |
| Wasser, Misc | add 100 | add 100 | add 100 | add 100 | add 100 |
| | Formel 31 | Formel 32 | Formel 33 | Formel 34 | Formel 35 |
| Polystyrolsulfonat | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Alkanolamin 3) | 80 bis 100 | 90 bis 100 | 95 bis 100 | 98 | 98 |
| Wasser, Misc | add 100 | add 100 | add 100 | add 100 | add 100 |
| | Formel 36 | Formel 37 | Formel 38 | Formel 39 | Formel 40 |
| Polystyrolsulfonat 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Alkanolamin 3) | 80 bis 100 | 90 bis 100 | 95 bis 100 | 98 | 98 |
| Wasser, Misc | add 100 | add 100 | add 100 | add 100 | add 100 |
| | Formel 41 | Formel 42 | Formel 43 | Formel 44 | Formel 45 |
| Polystyrolsulfonat 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| 2-Amino-2-methylpropanol 3) | 80 bis 100 | 90 bis 100 | 95 bis 100 | 98 | 98 |
| Wasser, Misc | add 100 | add 100 | add 100 | add 100 | add 100 |
| | Formel 46 | Formel 47 | Formel 48 | Formel 49 | Formel 50 |
| Polystyrolsulfonat 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Alkyltrimethylammoniumchlorid | 0,05 bis 3,0 | 0,1 bis 2,0 | 0,2 bis 1,0 | 0,5 | 0,3 |
| Wasser, Misc | add 100 | add 100 | add 100 | add 100 | add 100 |
| | Formel 51 | Formel 52 | Formel 53 | Formel 54 | Formel 55 |
| Polystyrolsulfonat 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Ölkörper | 0,01 bis 5,0 | 0,02 bis 4,0 | 0,05 bis 2,0 | 0,1 | 0,1 |
| Wasser, Misc | add 100 | add 100 | add 100 | add 100 | add 100 |
| | Formel 56 | Formel 57 | Formel 58 | Formel 59 | Formel 60 |
| Polystyrolsulfonat 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Wasser | 40 bis 99 | 50 bis 98 | 60 bis 95 | 97 | 92 |
| Misc | add 100 | add 100 | add 100 | add 100 | add 100 |
| | Formel 61 | Formel 62 | Formel 63 | Formel 64 | Formel 65 |
| Polystyrolsulfonat 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Alkyltrimethylammoniumchlorid | 0,05 bis 3,0 | 0,1 bis 2,0 | 0,2 bis 1,0 | 0,5 | 0,3 |
| Wasser | 40 bis 99 | 50 bis 98 | 60 bis 95 | 97 | 92 |
| Misc | add 100 | add 100 | add 100 | add 100 | add 100 |

(fortgesetzt)

| | Formel 66 | Formel 67 | Formel 68 | Formel 69 | Formel 70 |
|---|---|---|---|---|---|
| Polystyrolsulfonat 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Ölkörper | 0,01 bis 5,0 | 0,02 bis 4,0 | 0,05 bis 2,0 | 0,1 | 0,1 |
| Wasser | 40 bis 99 | 50 bis 98 | 60 bis 95 | 97 | 92 |
| Misc | add 100 | add 100 | add 100 | add 100 | add 100 |
| | Formel 71 | Formel 72 | Formel 73 | Formel 74 | Formel 75 |
| Polystyrolsulfonat 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| 2-Amino-2-methylpropanol 3) | 80 bis 100 | 90 bis 100 | 95 bis 100 | 98 | 98 |
| Ölkörper | 0,01 bis 5,0 | 0,02 bis 4,0 | 0,05 bis 2,0 | 0,1 | 0,1 |
| Wasser | 40 bis 99 | 50 bis 98 | 60 bis 95 | 97 | 92 |
| Misc | add 100 | add 100 | add 100 | add 100 | add 100 |

1) Polystyrolsulfonat mit einem Molukulargewicht von 80 bis 180 kDa
2) Copolymer B gebildet aus
- 3-Methyl-1-vinylimidazoliummethylsulfat (B1),
- N-Vinylpyrrolidon (B2),
- N-Vinylimidazol (B3) und
- Methacrylsäureamid (B4),
wobei das Copolymer B zu mindestens 95 Gew.-% aus den Struktureinheiten (B1), (B2), (B3) und (B4) besteht.
3) % der zur vollständigen Neutralisation des Polystyrolsulfonats benötigten Menge

Die Formulierung der erfindungsgemäßen Mittel kann in allen für kosmetische Mittel üblichen Formen erfolgen, beispielsweise in Form von Lösungen, die beispielsweise als Haarwasser auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind. In einer alternativen Ausführungsform können diese Mittel jedoch auch in Gel- oder in Cremeform vorliegen, wobei transparente Gele besonders bevorzugt sind.

Die erfindungsgemäßen Zusammensetzungen eignen sich insbesondere sehr gut dazu, Gasblasen im Mittel zu stabilisieren. Auf diese Weise können Luft oder andere Gase bzw. Gasgemische gut und langzeitstabil in die erfindungsgemäßen Mittel eingearbeitet werden. Dies kann wahlweise bei der Herstellung der Mittel geschehen, indem das Mittel vor der Abfüllung mit Gas, vorzugsweise Luft, beaufschlagt wird und ein Produkt abgefüllt wird, das sichtbare Gasblasen enthält. Bevorzugt liegen die erfindungsgemäßen Mittel in Form eines Schaums vor. Als Schaum wird dabei eine Zubereitung bezeichnet, die Gas gefüllte Bläschen aufweist, welche von flüssigen (flüssiger Schaum) oder festen (fester Schaum) Wänden umgeben sind. Bei den in der nachfolgenden Tabelle angeführten Zusammensetzungen handelt es sich vorzugsweise um feste Schäume. Die Dichte bevorzugter Zusammensetzungen beträgt 0,3 bis 1,0 g/$cm^3$, vorzugsweise 0,4 bis 0,9 g/$cm^3$ und insbesondere 0,5 bis 0,8 g/$cm^3$. Die Herstellung solcher Schäume kann beispielsweise durch Aufschlagen der Zubereitung in einem geeigneten Mischer oder durch Beaufschlagung mit einem geeigneten Gas, vorzugsweise Luft, erfolgen.

Die Zusammensetzung einiger bevorzugter kosmetischer Schäume kann der folgenden Tabellen entnommen werden. In dieser Tabelle verweist die linke Spalte ("Formel x") auf jeweils eine der in den weiter oben offenbarten Tabellen angeführten beispielhaften kosmetischen Zusammensetzungen. Die weiteren Spalten zwei bis sieben ("Dichte") geben jeweils die Dichte der entsprechenden kosmetischen Zusammensetzung an.

Eine kosmetische Zubereitung gemäß Zeile 12, Spalte 5 der nachfolgenden Tabelle, umfasst mit anderen Worten ein kosmetisches Mittel gemäß Formel 11 mit einer Dichte von 0,44 g/$cm^3$.

| | Dichte [g/$cm^3$] | | | | | |
|---|---|---|---|---|---|---|
| Formel 1 | 0,3 bis 1,0 | 0,93 | 0,4 bis 0,9 | 0,44 | 0,5 bis 0,8 | 0,61 |
| Formel 2 | 0,3 bis 1,0 | 0,93 | 0,4 bis 0,9 | 0,44 | 0,5 bis 0,8 | 0,61 |

(fortgesetzt)

| | Dichte [g/cm$^3$] | | | | | |
|---|---|---|---|---|---|---|
| Formel 3 | 0,3 bis 1,0 | 0,93 | 0,4 bis 0,9 | 0,44 | 0,5 bis 0,8 | 0,61 |
| Formel 4 | 0,3 bis 1,0 | 0,93 | 0,4 bis 0,9 | 0,44 | 0,5 bis 0,8 | 0,61 |
| Formel 5 | 0,3 bis 1,0 | 0,93 | 0,4 bis 0,9 | 0,44 | 0,5 bis 0,8 | 0,61 |
| Formel 6 | 0,3 bis 1,0 | 0,93 | 0,4 bis 0,9 | 0,44 | 0,5 bis 0,8 | 0,61 |
| Formel 7 | 0,3 bis 1,0 | 0,93 | 0,4 bis 0,9 | 0,44 | 0,5 bis 0,8 | 0,61 |
| Formel 8 | 0,3 bis 1,0 | 0,93 | 0,4 bis 0,9 | 0,44 | 0,5 bis 0,8 | 0,61 |
| Formel 9 | 0,3 bis 1,0 | 0,93 | 0,4 bis 0,9 | 0,44 | 0,5 bis 0,8 | 0,61 |
| Formel 10 | 0,3 bis 1,0 | 0,93 | 0,4 bis 0,9 | 0,44 | 0,5 bis 0,8 | 0,61 |
| Formel 11 | 0,3 bis 1,0 | 0,93 | 0,4 bis 0,9 | 0,44 | 0,5 bis 0,8 | 0,61 |
| Formel 12 | 0,3 bis 1,0 | 0,93 | 0,4 bis 0,9 | 0,44 | 0,5 bis 0,8 | 0,61 |
| Formel 13 | 0,3 bis 1,0 | 0,93 | 0,4 bis 0,9 | 0,44 | 0,5 bis 0,8 | 0,61 |
| Formel 14 | 0,3 bis 1,0 | 0,93 | 0,4 bis 0,9 | 0,44 | 0,5 bis 0,8 | 0,61 |
| Formel 15 | 0,3 bis 1,0 | 0,93 | 0,4 bis 0,9 | 0,44 | 0,5 bis 0,8 | 0,61 |
| Formel 16 | 0,3 bis 1,0 | 0,93 | 0,4 bis 0,9 | 0,44 | 0,5 bis 0,8 | 0,61 |
| Formel 17 | 0,3 bis 1,0 | 0,93 | 0,4 bis 0,9 | 0,44 | 0,5 bis 0,8 | 0,61 |
| Formel 18 | 0,3 bis 1,0 | 0,93 | 0,4 bis 0,9 | 0,44 | 0,5 bis 0,8 | 0,61 |
| Formel 19 | 0,3 bis 1,0 | 0,93 | 0,4 bis 0,9 | 0,44 | 0,5 bis 0,8 | 0,61 |
| Formel 20 | 0,3 bis 1,0 | 0,93 | 0,4 bis 0,9 | 0,44 | 0,5 bis 0,8 | 0,61 |
| Formel 21 | 0,3 bis 1,0 | 0,93 | 0,4 bis 0,9 | 0,44 | 0,5 bis 0,8 | 0,61 |
| Formel 22 | 0,3 bis 1,0 | 0,93 | 0,4 bis 0,9 | 0,44 | 0,5 bis 0,8 | 0,61 |
| Formel 23 | 0,3 bis 1,0 | 0,93 | 0,4 bis 0,9 | 0,44 | 0,5 bis 0,8 | 0,61 |
| Formel 24 | 0,3 bis 1,0 | 0,93 | 0,4 bis 0,9 | 0,44 | 0,5 bis 0,8 | 0,61 |
| Formel 25 | 0,3 bis 1,0 | 0,93 | 0,4 bis 0,9 | 0,44 | 0,5 bis 0,8 | 0,61 |
| Formel 26 | 0,3 bis 1,0 | 0,93 | 0,4 bis 0,9 | 0,44 | 0,5 bis 0,8 | 0,61 |
| Formel 27 | 0,3 bis 1,0 | 0,93 | 0,4 bis 0,9 | 0,44 | 0,5 bis 0,8 | 0,61 |
| Formel 28 | 0,3 bis 1,0 | 0,93 | 0,4 bis 0,9 | 0,44 | 0,5 bis 0,8 | 0,61 |
| Formel 29 | 0,3 bis 1,0 | 0,93 | 0,4 bis 0,9 | 0,44 | 0,5 bis 0,8 | 0,61 |
| Formel 30 | 0,3 bis 1,0 | 0,93 | 0,4 bis 0,9 | 0,44 | 0,5 bis 0,8 | 0,61 |
| Formel 31 | 0,3 bis 1,0 | 0,93 | 0,4 bis 0,9 | 0,44 | 0,5 bis 0,8 | 0,61 |
| Formel 32 | 0,3 bis 1,0 | 0,93 | 0,4 bis 0,9 | 0,44 | 0,5 bis 0,8 | 0,61 |
| Formel 33 | 0,3 bis 1,0 | 0,93 | 0,4 bis 0,9 | 0,44 | 0,5 bis 0,8 | 0,61 |
| Formel 34 | 0,3 bis 1,0 | 0,93 | 0,4 bis 0,9 | 0,44 | 0,5 bis 0,8 | 0,61 |
| Formel 35 | 0,3 bis 1,0 | 0,93 | 0,4 bis 0,9 | 0,44 | 0,5 bis 0,8 | 0,61 |
| Formel 36 | 0,3 bis 1,0 | 0,93 | 0,4 bis 0,9 | 0,44 | 0,5 bis 0,8 | 0,61 |
| Formel 37 | 0,3 bis 1,0 | 0,93 | 0,4 bis 0,9 | 0,44 | 0,5 bis 0,8 | 0,61 |
| Formel 38 | 0,3 bis 1,0 | 0,93 | 0,4 bis 0,9 | 0,44 | 0,5 bis 0,8 | 0,61 |
| Formel 39 | 0,3 bis 1,0 | 0,93 | 0,4 bis 0,9 | 0,44 | 0,5 bis 0,8 | 0,61 |
| Formel 40 | 0,3 bis 1,0 | 0,93 | 0,4 bis 0,9 | 0,44 | 0,5 bis 0,8 | 0,61 |

(fortgesetzt)

| | Dichte [g/cm³] | | | | | |
|---|---|---|---|---|---|---|
| Formel 41 | 0,3 bis 1,0 | 0,93 | 0,4 bis 0,9 | 0,44 | 0,5 bis 0,8 | 0,61 |
| Formel 42 | 0,3 bis 1,0 | 0,93 | 0,4 bis 0,9 | 0,44 | 0,5 bis 0,8 | 0,61 |
| Formel 43 | 0,3 bis 1,0 | 0,93 | 0,4 bis 0,9 | 0,44 | 0,5 bis 0,8 | 0,61 |
| Formel 44 | 0,3 bis 1,0 | 0,93 | 0,4 bis 0,9 | 0,44 | 0,5 bis 0,8 | 0,61 |
| Formel 45 | 0,3 bis 1,0 | 0,93 | 0,4 bis 0,9 | 0,44 | 0,5 bis 0,8 | 0,61 |
| Formel 46 | 0,3 bis 1,0 | 0,93 | 0,4 bis 0,9 | 0,44 | 0,5 bis 0,8 | 0,61 |
| Formel 47 | 0,3 bis 1,0 | 0,93 | 0,4 bis 0,9 | 0,44 | 0,5 bis 0,8 | 0,61 |
| Formel 48 | 0,3 bis 1,0 | 0,93 | 0,4 bis 0,9 | 0,44 | 0,5 bis 0,8 | 0,61 |
| Formel 49 | 0,3 bis 1,0 | 0,93 | 0,4 bis 0,9 | 0,44 | 0,5 bis 0,8 | 0,61 |
| Formel 50 | 0,3 bis 1,0 | 0,93 | 0,4 bis 0,9 | 0,44 | 0,5 bis 0,8 | 0,61 |
| Formel 51 | 0,3 bis 1,0 | 0,93 | 0,4 bis 0,9 | 0,44 | 0,5 bis 0,8 | 0,61 |
| Formel 52 | 0,3 bis 1,0 | 0,93 | 0,4 bis 0,9 | 0,44 | 0,5 bis 0,8 | 0,61 |
| Formel 53 | 0,3 bis 1,0 | 0,93 | 0,4 bis 0,9 | 0,44 | 0,5 bis 0,8 | 0,61 |
| Formel 54 | 0,3 bis 1,0 | 0,93 | 0,4 bis 0,9 | 0,44 | 0,5 bis 0,8 | 0,61 |
| Formel 55 | 0,3 bis 1,0 | 0,93 | 0,4 bis 0,9 | 0,44 | 0,5 bis 0,8 | 0,61 |
| Formel 56 | 0,3 bis 1,0 | 0,93 | 0,4 bis 0,9 | 0,44 | 0,5 bis 0,8 | 0,61 |
| Formel 57 | 0,3 bis 1,0 | 0,93 | 0,4 bis 0,9 | 0,44 | 0,5 bis 0,8 | 0,61 |
| Formel 58 | 0,3 bis 1,0 | 0,93 | 0,4 bis 0,9 | 0,44 | 0,5 bis 0,8 | 0,61 |
| Formel 59 | 0,3 bis 1,0 | 0,93 | 0,4 bis 0,9 | 0,44 | 0,5 bis 0,8 | 0,61 |
| Formel 60 | 0,3 bis 1,0 | 0,93 | 0,4 bis 0,9 | 0,44 | 0,5 bis 0,8 | 0,61 |
| Formel 61 | 0,3 bis 1,0 | 0,93 | 0,4 bis 0,9 | 0,44 | 0,5 bis 0,8 | 0,61 |
| Formel 62 | 0,3 bis 1,0 | 0,93 | 0,4 bis 0,9 | 0,44 | 0,5 bis 0,8 | 0,61 |
| Formel 63 | 0,3 bis 1,0 | 0,93 | 0,4 bis 0,9 | 0,44 | 0,5 bis 0,8 | 0,61 |
| Formel 64 | 0,3 bis 1,0 | 0,93 | 0,4 bis 0,9 | 0,44 | 0,5 bis 0,8 | 0,61 |
| Formel 65 | 0,3 bis 1,0 | 0,93 | 0,4 bis 0,9 | 0,44 | 0,5 bis 0,8 | 0,61 |
| Formel 66 | 0,3 bis 1,0 | 0,93 | 0,4 bis 0,9 | 0,44 | 0,5 bis 0,8 | 0,61 |
| Formel 67 | 0,3 bis 1,0 | 0,93 | 0,4 bis 0,9 | 0,44 | 0,5 bis 0,8 | 0,61 |
| Formel 68 | 0,3 bis 1,0 | 0,93 | 0,4 bis 0,9 | 0,44 | 0,5 bis 0,8 | 0,61 |
| Formel 69 | 0,3 bis 1,0 | 0,93 | 0,4 bis 0,9 | 0,44 | 0,5 bis 0,8 | 0,61 |
| Formel 70 | 0,3 bis 1,0 | 0,93 | 0,4 bis 0,9 | 0,44 | 0,5 bis 0,8 | 0,61 |
| Formel 71 | 0,3 bis 1,0 | 0,93 | 0,4 bis 0,9 | 0,44 | 0,5 bis 0,8 | 0,61 |
| Formel 72 | 0,3 bis 1,0 | 0,93 | 0,4 bis 0,9 | 0,44 | 0,5 bis 0,8 | 0,61 |
| Formel 73 | 0,3 bis 1,0 | 0,93 | 0,4 bis 0,9 | 0,44 | 0,5 bis 0,8 | 0,61 |
| Formel 74 | 0,3 bis 1,0 | 0,93 | 0,4 bis 0,9 | 0,44 | 0,5 bis 0,8 | 0,61 |
| Formel 75 | 0,3 bis 1,0 | 0,93 | 0,4 bis 0,9 | 0,44 | 0,5 bis 0,8 | 0,61 |

In einer alternativen Ausführungsform können die erfindungsgemäßen kosmetischen Mittel als Pump- oder Aerosolspray konfektioniert werden. Bevorzugte Aerosolsprays enthalten dabei neben weiteren Aktiv- und Hilfsstoffen ein Treibmittel. Erfindungsgemäß geeignete Treibmittel (Treibgase) sind Propan, n-Butan, iso-Butan, Dimethylether

(DME), Stickstoff, Luft, Lachgas, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Dimethylether, Propan, n-Butan, iso-Butan sowie Mischungen dieser Treibgase. Ganz besonders bevorzugt ist der Einsatz von Propan/Butan Gemischen oder Isobutan. Kosmetische Mittel, die das Treibmittel bezogen auf ihr Gesamtgewicht in einer Menge von 2,0 - 20 Gew.%, bevorzugt 4,0 - 15 Gew.% und besonders bevorzugt 5,0 - 10 Gew.-% enthalten, sind erfindungsgemäß bevorzugt.

Die Zusammensetzung einiger bevorzugter Treibmittel-haltiger kosmetischer Mittel kann der folgenden Tabellen entnommen werden. In dieser Tabelle verweist die linke Spalte ("Formel x") auf jeweils eine der in den weiter oben offenbarten Tabellen angeführten beispielhaften kosmetischen Zusammensetzungen. Die weiteren Spalten zwei bis sieben ("Treibmittel") geben jeweils die der entsprechenden kosmetischen Zusammensetzung zugesetzte Menge an Treibmittel an. Diese Angaben in "Gew.-%" beziehen sich auf das Gesamtgewicht der kosmetischen Zusammensetzung der jeweiligen "Formel x" ohne Treibmittel.

Eine kosmetische Zubereitung gemäß Zeile 12, Spalte 5 der nachfolgenden Tabelle, umfasst mit anderen Worten eine 20:1 Mischung des Treibmittel-freien kosmetischen Mittels gemäß Formel 11 mit einem Propan/Butan Gemisch.

| | Treibmittel [Gew.-%] | | | | | |
|---|---|---|---|---|---|---|
| Formel 1 | 2,0 bis 10 | 5,0 | 2,0 bis 10 P/B* | 5,0 P/B | 2,0 bis 10 iB* | 5,0 iB |
| Formel 2 | 2,0 bis 10 | 5,0 | 2,0 bis 10 P/B* | 5,0 P/B | 2,0 bis 10 iB* | 5,0 iB |
| Formel 3 | 2,0 bis 10 | 5,0 | 2,0 bis 10 P/B* | 5,0 P/B | 2,0 bis 10 iB* | 5,0 iB |
| Formel 4 | 2,0 bis 10 | 5,0 | 2,0 bis 10 P/B* | 5,0 P/B | 2,0 bis 10 iB* | 5,0 iB |
| Formel 5 | 2,0 bis 10 | 5,0 | 2,0 bis 10 P/B* | 5,0 P/B | 2,0 bis 10 iB* | 5,0 iB |
| Formel 6 | 2,0 bis 10 | 5,0 | 2,0 bis 10 P/B* | 5,0 P/B | 2,0 bis 10 iB* | 5,0 iB |
| Formel 7 | 2,0 bis 10 | 5,0 | 2,0 bis 10 P/B* | 5,0 P/B | 2,0 bis 10 iB* | 5,0 iB |
| Formel 8 | 2,0 bis 10 | 5,0 | 2,0 bis 10 P/B* | 5,0 P/B | 2,0 bis 10 iB* | 5,0 iB |
| Formel 9 | 2,0 bis 10 | 5,0 | 2,0 bis 10 P/B* | 5,0 P/B | 2,0 bis 10 iB* | 5,0 iB |
| Formel 10 | 2,0 bis 10 | 5,0 | 2,0 bis 10 P/B* | 5,0 P/B | 2,0 bis 10 iB* | 5,0 iB |
| Formel 11 | 2,0 bis 10 | 5,0 | 2,0 bis 10 P/B* | 5,0 P/B | 2,0 bis 10 iB* | 5,0 iB |
| Formel 12 | 2,0 bis 10 | 5,0 | 2,0 bis 10 P/B* | 5,0 P/B | 2,0 bis 10 iB* | 5,0 iB |
| Formel 13 | 2,0 bis 10 | 5,0 | 2,0 bis 10 P/B* | 5,0 P/B | 2,0 bis 10 iB* | 5,0 iB |
| Formel 14 | 2,0 bis 10 | 5,0 | 2,0 bis 10 P/B* | 5,0 P/B | 2,0 bis 10 iB* | 5,0 iB |
| Formel 15 | 2,0 bis 10 | 5,0 | 2,0 bis 10 P/B* | 5,0 P/B | 2,0 bis 10 iB* | 5,0 iB |
| Formel 16 | 2,0 bis 10 | 5,0 | 2,0 bis 10 P/B* | 5,0 P/B | 2,0 bis 10 iB* | 5,0 iB |
| Formel 17 | 2,0 bis 10 | 5,0 | 2,0 bis 10 P/B* | 5,0 P/B | 2,0 bis 10 iB* | 5,0 iB |
| Formel 18 | 2,0 bis 10 | 5,0 | 2,0 bis 10 P/B* | 5,0 P/B | 2,0 bis 10 iB* | 5,0 iB |
| Formel 19 | 2,0 bis 10 | 5,0 | 2,0 bis 10 P/B* | 5,0 P/B | 2,0 bis 10 iB* | 5,0 iB |
| Formel 20 | 2,0 bis 10 | 5,0 | 2,0 bis 10 P/B* | 5,0 P/B | 2,0 bis 10 iB* | 5,0 iB |
| Formel 21 | 2,0 bis 10 | 5,0 | 2,0 bis 10 P/B* | 5,0 P/B | 2,0 bis 10 iB* | 5,0 iB |
| Formel 22 | 2,0 bis 10 | 5,0 | 2,0 bis 10 P/B* | 5,0 P/B | 2,0 bis 10 iB* | 5,0 iB |
| Formel 23 | 2,0 bis 10 | 5,0 | 2,0 bis 10 P/B* | 5,0 P/B | 2,0 bis 10 iB* | 5,0 iB |
| Formel 24 | 2,0 bis 10 | 5,0 | 2,0 bis 10 P/B* | 5,0 P/B | 2,0 bis 10 iB* | 5,0 iB |
| Formel 25 | 2,0 bis 10 | 5,0 | 2,0 bis 10 P/B* | 5,0 P/B | 2,0 bis 10 iB* | 5,0 iB |
| Formel 26 | 2,0 bis 10 | 5,0 | 2,0 bis 10 P/B* | 5,0 P/B | 2,0 bis 10 iB* | 5,0 iB |
| Formel 27 | 2,0 bis 10 | 5,0 | 2,0 bis 10 P/B* | 5,0 P/B | 2,0 bis 10 iB* | 5,0 iB |

(fortgesetzt)

| | Treibmittel [Gew.-%] | | | | | |
|---|---|---|---|---|---|---|
| Formel 28 | 2,0 bis 10 | 5,0 | 2,0 bis 10 P/B* | 5,0 P/B | 2,0 bis 10 iB* | 5,0 iB |
| Formel 29 | 2,0 bis 10 | 5,0 | 2,0 bis 10 P/B* | 5,0 P/B | 2,0 bis 10 iB* | 5,0 iB |
| Formel 30 | 2,0 bis 10 | 5,0 | 2,0 bis 10 P/B* | 5,0 P/B | 2,0 bis 10 iB* | 5,0 iB |
| Formel 31 | 2,0 bis 10 | 5,0 | 2,0 bis 10 P/B* | 5,0 P/B | 2,0 bis 10 iB* | 5,0 iB |
| Formel 32 | 2,0 bis 10 | 5,0 | 2,0 bis 10 P/B* | 5,0 P/B | 2,0 bis 10 iB* | 5,0 iB |
| Formel 33 | 2,0 bis 10 | 5,0 | 2,0 bis 10 P/B* | 5,0 P/B | 2,0 bis 10 iB* | 5,0 iB |
| Formel 34 | 2,0 bis 10 | 5,0 | 2,0 bis 10 P/B* | 5,0 P/B | 2,0 bis 10 iB* | 5,0 iB |
| Formel 35 | 2,0 bis 10 | 5,0 | 2,0 bis 10 P/B* | 5,0 P/B | 2,0 bis 10 iB* | 5,0 iB |
| Formel 36 | 2,0 bis 10 | 5,0 | 2,0 bis 10 P/B* | 5,0 P/B | 2,0 bis 10 iB* | 5,0 iB |
| Formel 37 | 2,0 bis 10 | 5,0 | 2,0 bis 10 P/B* | 5,0 P/B | 2,0 bis 10 iB* | 5,0 iB |
| Formel 38 | 2,0 bis 10 | 5,0 | 2,0 bis 10 P/B* | 5,0 P/B | 2,0 bis 10 iB* | 5,0 iB |
| Formel 39 | 2,0 bis 10 | 5,0 | 2,0 bis 10 P/B* | 5,0 P/B | 2,0 bis 10 iB* | 5,0 iB |
| Formel 40 | 2,0 bis 10 | 5,0 | 2,0 bis 10 P/B* | 5,0 P/B | 2,0 bis 10 iB* | 5,0 iB |
| Formel 41 | 2,0 bis 10 | 5,0 | 2,0 bis 10 P/B* | 5,0 P/B | 2,0 bis 10 iB* | 5,0 iB |
| Formel 42 | 2,0 bis 10 | 5,0 | 2,0 bis 10 P/B* | 5,0 P/B | 2,0 bis 10 iB* | 5,0 iB |
| Formel 43 | 2,0 bis 10 | 5,0 | 2,0 bis 10 P/B* | 5,0 P/B | 2,0 bis 10 iB* | 5,0 iB |
| Formel 44 | 2,0 bis 10 | 5,0 | 2,0 bis 10 P/B* | 5,0 P/B | 2,0 bis 10 iB* | 5,0 iB |
| Formel 45 | 2,0 bis 10 | 5,0 | 2,0 bis 10 P/B* | 5,0 P/B | 2,0 bis 10 iB* | 5,0 iB |
| Formel 46 | 2,0 bis 10 | 5,0 | 2,0 bis 10 P/B* | 5,0 P/B | 2,0 bis 10 iB* | 5,0 iB |
| Formel 47 | 2,0 bis 10 | 5,0 | 2,0 bis 10 P/B* | 5,0 P/B | 2,0 bis 10 iB* | 5,0 iB |
| Formel 48 | 2,0 bis 10 | 5,0 | 2,0 bis 10 P/B* | 5,0 P/B | 2,0 bis 10 iB* | 5,0 iB |
| Formel 49 | 2,0 bis 10 | 5,0 | 2,0 bis 10 P/B* | 5,0 P/B | 2,0 bis 10 iB* | 5,0 iB |
| Formel 50 | 2,0 bis 10 | 5,0 | 2,0 bis 10 P/B* | 5,0 P/B | 2,0 bis 10 iB* | 5,0 iB |
| Formel 51 | 2,0 bis 10 | 5,0 | 2,0 bis 10 P/B* | 5,0 P/B | 2,0 bis 10 iB* | 5,0 iB |
| Formel 52 | 2,0 bis 10 | 5,0 | 2,0 bis 10 P/B* | 5,0 P/B | 2,0 bis 10 iB* | 5,0 iB |
| Formel 53 | 2,0 bis 10 | 5,0 | 2,0 bis 10 P/B* | 5,0 P/B | 2,0 bis 10 iB* | 5,0 iB |
| Formel 54 | 2,0 bis 10 | 5,0 | 2,0 bis 10 P/B* | 5,0 P/B | 2,0 bis 10 iB* | 5,0 iB |
| Formel 55 | 2,0 bis 10 | 5,0 | 2,0 bis 10 P/B* | 5,0 P/B | 2,0 bis 10 iB* | 5,0 iB |
| Formel 56 | 2,0 bis 10 | 5,0 | 2,0 bis 10 P/B* | 5,0 P/B | 2,0 bis 10 iB* | 5,0 iB |
| Formel 57 | 2,0 bis 10 | 5,0 | 2,0 bis 10 P/B* | 5,0 P/B | 2,0 bis 10 iB* | 5,0 iB |
| Formel 58 | 2,0 bis 10 | 5,0 | 2,0 bis 10 P/B* | 5,0 P/B | 2,0 bis 10 iB* | 5,0 iB |
| Formel 59 | 2,0 bis 10 | 5,0 | 2,0 bis 10 P/B* | 5,0 P/B | 2,0 bis 10 iB* | 5,0 iB |
| Formel 60 | 2,0 bis 10 | 5,0 | 2,0 bis 10 P/B* | 5,0 P/B | 2,0 bis 10 iB* | 5,0 iB |
| Formel 61 | 2,0 bis 10 | 5,0 | 2,0 bis 10 P/B* | 5,0 P/B | 2,0 bis 10 iB* | 5,0 iB |
| Formel 62 | 2,0 bis 10 | 5,0 | 2,0 bis 10 P/B* | 5,0 P/B | 2,0 bis 10 iB* | 5,0 iB |
| Formel 63 | 2,0 bis 10 | 5,0 | 2,0 bis 10 P/B* | 5,0 P/B | 2,0 bis 10 iB* | 5,0 iB |
| Formel 64 | 2,0 bis 10 | 5,0 | 2,0 bis 10 P/B* | 5,0 P/B | 2,0 bis 10 iB* | 5,0 iB |
| Formel 65 | 2,0 bis 10 | 5,0 | 2,0 bis 10 P/B* | 5,0 P/B | 2,0 bis 10 iB* | 5,0 iB |

(fortgesetzt)

| | Treibmittel [Gew.-%] | | | | | |
|---|---|---|---|---|---|---|
| Formel 66 | 2,0 bis 10 | 5,0 | 2,0 bis 10 P/B* | 5,0 P/B | 2,0 bis 10 iB* | 5,0 iB |
| Formel 67 | 2,0 bis 10 | 5,0 | 2,0 bis 10 P/B* | 5,0 P/B | 2,0 bis 10 iB* | 5,0 iB |
| Formel 68 | 2,0 bis 10 | 5,0 | 2,0 bis 10 P/B* | 5,0 P/B | 2,0 bis 10 iB* | 5,0 iB |
| Formel 69 | 2,0 bis 10 | 5,0 | 2,0 bis 10 P/B* | 5,0 P/B | 2,0 bis 10 iB* | 5,0 iB |
| Formel 70 | 2,0 bis 10 | 5,0 | 2,0 bis 10 P/B* | 5,0 P/B | 2,0 bis 10 iB* | 5,0 iB |
| Formel 71 | 2,0 bis 10 | 5,0 | 2,0 bis 10 P/B* | 5,0 P/B | 2,0 bis 10 iB* | 5,0 iB |
| Formel 72 | 2,0 bis 10 | 5,0 | 2,0 bis 10 P/B* | 5,0 P/B | 2,0 bis 10 iB* | 5,0 iB |
| Formel 73 | 2,0 bis 10 | 5,0 | 2,0 bis 10 P/B* | 5,0 P/B | 2,0 bis 10 iB* | 5,0 iB |
| Formel 74 | 2,0 bis 10 | 5,0 | 2,0 bis 10 P/B* | 5,0 P/B | 2,0 bis 10 iB* | 5,0 iB |
| Formel 75 | 2,0 bis 10 | 5,0 | 2,0 bis 10 P/B* | 5,0 P/B | 2,0 bis 10 iB* | 5,0 iB |
| * "P/B" entspricht einem Propan/Butan Gemisch<br>** "iB" entspricht Isobutan | | | | | | |

Wie eingangs ausgeführt, weisen die erfindungsgemäßen Mittel vorteilhafte haarfixierende Eigenschaften auf. Ein Verfahren zur temporären Verformung keratinischer Fasern, bei welchem eine erfindungsgemäße Zusammensetzung auf die keratinische Faser aufgebracht wird, ist daher ein weiterer Gegenstand der vorliegenden Anmeldung. Die Verwendung eines erfindungsgemäßen kosmetischen Mittels zur temporären Verformung keratinischer Fasern ist ein weiterer Gegenstand der vorliegenden Anmeldung. Wie eingangs ausgeführt, zeichnen sich die erfindungsgemäßen Mittel insbesondere durch einen verbesserten Halt bei der temporären Verformung keratinischer Fasern aus. Ein zusätzlicher Gegenstand der vorliegenden Anmeldung ist daher die Verwendung eines erfindungsgemäßen kosmetischen Mittels zur Verbesserung des Halts bei der temporären Verformung keratinischer Fasern.

Beispiele

Es wurde die Feuchtebeständigkeit (High Humidity Curl Retention; HHCR) der mittels der nachfolgenden drei haarkosmetischen Mittel erzielten temporären Haarverformung bestimmt.

| | **E1** | **V1** | **V2** |
|---|---|---|---|
| Flexan II[1] | 0,75 | 1,25 | -- |
| Luviquat Supreme [2] | 3,75 | -- | 6,25 |
| Natriumbenzoat | 0,3 | 0,3 | 0,3 |
| D-Panthenol (75%) | 0,2 | 0,2 | 0,2 |
| Dow Corning 939 [3] | 0,2 | 0,2 | 0,2 |
| Dehyquart A CA [4] | 1,0 | 1,0 | 1,0 |
| Castor Oil hydrogenated, 40 EO | 0,2 | 0,2 | 0,2 |
| Parfüm | 0,1 | 0,1 | 0,1 |
| Wasser, Misc | add 100 | add 100 | add 100 |
| [1]Polystyrolsulfonat (INCl: Sodium Polystyrene Sulfonate, 100%)<br>[2] N-Methylvinylimidazol/Vinylpyrrolidon/Vinylimidazol/Methacrylamid-Copolymer (INCI: Polyquaternium-68, 20% in Wasser)<br>[3] Silikon Zubereitung (INCI: Amodimethicone, Trideceth-12, Cetrimonium Chloride)<br>[4] Trimethylhexadecylammoniumchlorid | | | |

Zur Bestimmung der High Humidity Curl Retention wurden standardisierte Haarsträhnen der Fa. Kerling (Art.

Nr. 827560) des Haartyps "European Natural, Farbe 6/0) von einer Länge ($L_{max}$) von 220 mm und einem Gewicht von 0.6 g eingesetzt. Zur Vorbereitung wurden die Strähnen mit einer 12.5 Gew.-%igen Natriumlaurethsulphat-Lösung gewaschen. Die Haarsträhnen wurden über Nacht in einem Trockenofen bei 318 K getrocknet.

0.18 g der Zusammensetzungen wurden auf jeweils eine Haarsträhne appliziert und einmassiert. Die Strähne wurde sodann auf einen Wickler gewickelt (Fripac-medis, Ø 7mm, Art. Nr. D-1203) und über Nacht bei Raumtemperatur getrocknet.

Die Wickler wurden vorsichtig entfernt und die Strähne aufgehängt. Die Längen der Locken wurden jeweils gemessen ($L_0$) und die Strähnen in eine Klimakammer gegeben. Dort wurden sie bei 294 K und einer relativen Luftfeuchtigkeit von 85% über einen Zeitraum von 6 h gelagert und danach die Längen der Locken erneut vermessen ($L_t$).

Pro Zusammensetzung wurden 5 Teststrähnen entsprechend behandelt und vermessen.

Die High-Humidity-Curlretention (HHCR) wurde nach folgender Formel berechnet und für jede Zusammensetzung das arithmetische Mittel aus den HHCR-Werten der 5 Teststrähnen gebildet:

$$HHCR = \frac{L_{\max} - L_t}{L_{\max} - L_0}$$

| | E1 | V1 | V2 |
|---|---|---|---|
| HHCR | 89% | 49% | 61% |

Den Messdaten ist die synergistische Wirkung der erfindungsgemäßen Polymerkombination in Zusammensetzung E1 zu entnehmen.

**Patentansprüche**

1. Kosmetisches Mittel, enthaltend in einem kosmetisch akzeptablen Träger

   a) mindestens ein Polystyrolsulfonat
   b) mindestens ein Copolymer B gebildet aus

      - mindestens einem Monomer (B1) ausgewählt aus quaternisierten N-Vinylimidazolen,
      - mindestens einem Monomer (B2) ausgewählt aus N-Vinyllactamen,

   **dadurch gekennzeichnet, dass** das Gewichtsverhältnis von Polystryolsulfonat und Copolymer B zwischen 4:1 und 1:4 beträgt.

2. Kosmetisches Mittel nach Anspruch 1, **dadurch gekennzeichnet, dass** das Polystyrolsulfonat ein Molekulargewicht von 80 bis 180 kDa, vorzugsweise von 100 bis 160 kDa und insbesondere von 120 bis 140 kDa aufweist.

3. Kosmetisches Mittel nach einem der vorherigen Ansprüche, **dadurch gekennzeichnet, dass** der Gewichtsanteil des Polystyrolsulfonats A am Gesamtgewicht des Mittels 0,05 bis 10 Gew.-%, vorzugsweise 0,1 bis 7,0 Gew.-% und insbesondere 0,2 bis 5,0 Gew.-% beträgt.

4. Kosmetisches Mittel nach einem der vorherigen Ansprüche, **dadurch gekennzeichnet, dass** das Copolymer B gebildet ist aus

   - 3-Methyl-1-vinylimidazoliummethylsulfat (B1),
   - mindestens einem Monomer (B2) ausgewählt aus N-Vinylcaprolactam und N-Vinylpyrrolidon,
   - N-Vinylimidazol (B3) und
   - mindestens einem Monomer (B4) ausgewählt aus Acrylsäureamid und Methacrylsäureamid.

5. Kosmetisches Mittel nach einem der vorherigen Ansprüche, **dadurch gekennzeichnet, dass** der Gewichtsanteil des Copolymers B am Gesamtgewicht des Mittels 0,05 bis 10 Gew.-%, vorzugsweise 0,1 bis 7,0 Gew.-% und insbesondere 0,2 bis 5,0 Gew.-% beträgt.

6. Kosmetisches Mittel nach einem der vorherigen Ansprüche, **dadurch gekennzeichnet, dass** weiterhin mindestens eine monomere quartäre Ammoniumverbindung, vorzugsweise eine monomere quartäre Ammoniumverbindung ausgewählt aus der Gruppe der Trimethylalkylammoniumhalogenide enthalten ist.

7. Kosmetisches Mittel nach einem der vorherigen Ansprüche, **dadurch gekennzeichnet, dass** es weiterhin mindestens einen Ölkörper, vorzugsweise mindestens einen Ölkörper aus der Gruppe der Silikonöle, enthält.

8. Verwendung eines kosmetischen Mittels nach einem der vorherigen Ansprüche, zur temporären Verformung keratinischer Fasern.

9. Verfahren zur temporären Verformung keratinischer Fasern, bei welchem eine Zusammensetzung nach einem der Ansprüche 1 bis 7 auf die keratinische Faser aufgebracht wird.

## Claims

1. A cosmetic agent containing, in a cosmetically acceptable carrier,

   a) at least one polystyrene sulfonate
   b) at least one copolymer B formed of

      - at least one monomer (B1) selected from quaternized N-vinylimidazoles,
      - at least one monomer (B2) selected from N-vinyl lactams,

   **characterized in that** the weight ratio of polystyrene sulfonate and copolymer B is between 4:1 and 1:4.

2. The cosmetic agent according to claim 1, **characterized in that** the polystyrene sulfonate has a molecular weight of from 80 to 180 kDa, preferably 100 to 160 kDa and in particular 120 to 140 kDa.

3. The cosmetic agent according to one of the preceding claims, **characterized in that** the weight proportion of the polystyrene sulfonate A to the total weight of the agent is 0.05 to 10 wt.%, preferably 0.1 to 7.0 wt.% and in particular 0.2 to 5.0 wt.%.

4. The cosmetic agent according to one of the preceding claims, **characterized in that** the copolymer B is formed of

   - 3-methyl-1-vinylimidazolium methyl sulfate (B1),
   - at least one monomer (B2) selected from N-vinyl caprolactam and N-vinylpyrrolidone,
   - N-vinylimidazole (B3) and
   - at least one monomer (B4) selected from acrylic acid amide and methacrylic acid amide.

5. The cosmetic agent according to one of the preceding claims, **characterized in that** the weight proportion of the copolymer B to the total weight of the agent is 0.05 to 10 wt.%, preferably 0.1 to 7.0 wt.% and in particular 0.2 to 5.0 wt.%.

6. The cosmetic agent according to one of the preceding claims, **characterized in that** additionally at least one monomeric quaternary ammonium compound, preferably a monomeric quaternary ammonium compound selected from the group of trimethyl alkyl ammonium halides, is contained.

7. The cosmetic agent according to one of the preceding claims, **characterized in that** it additionally contains at least one oleosome, preferably at least one oleosome from the group of silicone oils.

8. The use of a cosmetic agent according to one of the preceding claims, for temporary shaping of keratin fibers.

9. A method for temporary shaping of keratin fibers, in which a composition according to one of claims 1 to 7 is applied to the keratin fibers.

## Revendications

1. Agent cosmétique contenant, dans un support cosmétiquement a cceptable,

   a) au moins un polystyrène sulfonate
   b) au moins un copolymère B formé à partir de

      - au moins un monomère (B1) choisi parmi les N-vinylimidazoles quaternisés,
      - au moins un monomère (B2) choisi parmi les N-vinyllactames,

   **caractérisé en ce que** le rapport en poids entre le polystyrène sulfonate et le copolymère B est compris entre 4:1 et 1:4.

2. Agent cosmétique selon la revendication 1, **caractérisé en ce que** le polystyrène sulfonate a un poids moléculaire de 80 à 180 kDa, de préférence de 100 à 160 kDa et en particulier de 120 à 140 kDa.

3. Agent cosmétique selon l'une des revendications précédentes, **caractérisé en ce que** la proportion en poids du polystyrène sulfonate A sur le poids total de l'agent est de 0,05 à 10 % en poids, de préférence de 0,1 à 7,0 % en poids et en particulier de 0,2 à 5,0 % en poids.

4. Agent cosmétique selon l'une des revendications précédentes, **caractérisé en ce que** le copolymère B est formé à partir de

   - méthylsulfate de 3-méthyl-1-vinylimidazolium (B1),
   - au moins un monomère (B2) choisi parmi le N-vinylcaprolactame et la N-vinylpyrrolidone,
   - N-vinylimidazole (B3) et
   - au moins un monomère (B4) choisi parmi l'acrylamide et le méthacrylamide.

5. Agent cosmétique selon l'une des revendications précédentes, **caractérisé en ce que** la proportion en poids du copolymère B au poids total de l'agent est de 0,05 à 10 % en poids, de préférence de 0,1 à 7,0 % en poids et en particulier de 0,2 à 5,0 % en poids.

6. Agent cosmétique selon l'une des revendications précédentes, **caractérisé en ce qu'**il contient en outre au moins un composé d'ammonium quaternaire monomère, de préférence un composé d'ammonium quaternaire monomère choisi dans le groupe des halogénures de triméthylalkylammonium.

7. Agent cosmétique selon l'une des revendications précédentes, **caractérisé en ce qu'**il contient en outre au moins un corps huileux, de préférence au moins un corps huileux du groupe des huiles siliconées.

8. Utilisation d'un agent cosmétique selon l'une des revendications précédentes pour la mise en forme temporaire des fibres de kératine.

9. Procédé de mise en forme temporaire de fibres de kératine dans lequel une composition selon l'une des revendications 1 à 7 est appliquée sur la fibre de kératine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102006045964 A1
- EP 2335673 A1